# EUROPEAN PATENT APPLICATION

(11) **EP 4 029 528 A1**
(43) Date of publication of application: **20.07.2022**
(21) Application number: 21151291.8
(22) Date of filing: 13.01.2021
(51) Int. Cl.: A61L 2/10, E05B 1/00

(54) **EXTERIOR SURFACE STRUCTURE OF A RADIATION EXIT WINDOW**

(71) Applicant: Koninklijke Philips N.V., 5656 AG Eindhoven (NL)
(72) Inventor: Hietbrink, Roelant Boudewijn, 5656 AE Eindhoven (NL); Salters, Bart Andre, 5656 AE Eindhoven (NL); NIESSEN, Eduard Matheus Johannes, 5656 AE Eindhoven (NL); Martens, Peter, 5656 AE Eindhoven (NL)
(74) Representative: Philips Intellectual Property & Standards

(57) **Abstract**

In a system (1) comprising a radiation body (10) configured to receive radiation that at least comprises UV radiation (22), at least one area (12) of a radiation exit window (11) of the radiation body (10) is prevented from receiving the UV radiation (22) at an intensity that is equal to or higher than a disinfecting minimum intensity. In order to avoid that a health risk arises from having the at least one insufficiently irradiated area (12), the radiation exit window (11) is provided with an exterior surface structure that is configured to prevent contact between a body part (5) of a human being or higher animal and the radiation exit window (11) at the position of the at least one insufficiently irradiated area (12) when the human being or higher animal touches the radiation exit window (11) through the body part (5).

## Description

### FIELD OF THE INVENTION

The invention relates to a system comprising a radiation body comprising a radiation exit window, wherein the radiation body is configured to receive radiation that at least comprises UV radiation, and wherein the radiation exit window is capable of allowing at least part of the radiation to pass to the exterior of the radiation body.

Further, the invention relates to an object comprising the system as described here before, wherein the radiation exit window of the radiation body of the system is arranged to be at an exterior side of the object.

Still further, the invention relates to a method of adding a system as described here before to an existing object, wherein the radiation body of the system is applied to an exterior surface of the existing object.

### BACKGROUND OF THE INVENTION

WO 2018/215272 A1 discloses a system comprising a waveguide element that is configured to function as the above-mentioned radiation body, and at least one UV radiation source. WO 2018/215272 A1 teaches that the UV radiation emitted by the at least one UV radiation source is used for killing microorganisms as may be present on the radiation exit window, or for rendering the microorganisms inactive or unable to reproduce. During operation of the known system, UV radiation is coupled out of the waveguide element through the radiation exit window at the very positions where microorganisms are present on the window. As a practical example of microorganisms, bacteria are mentioned.

Among other things, the invention that is the subject of WO 2018/215272 A1 provides an object comprising the system, wherein the object comprises an external surface, and wherein the radiation exit window of the waveguide element of the system is configured as at least part of the external surface. Examples of such an object include a door knob, a tap knob, a toilet knob, a seating of a toilet, a railing, a kitchen cutting board, a kitchen wall, a table, or (other) common (household) objects, i.e. objects which are especially designed to be used at home or in offices, etc. Further examples of such an object include a cleanroom wall and medical devices such as an operating table and an operation room wall. In the context of all of such possible practical applications, it is important that external surfaces are kept in a disinfected state.

Generally speaking, applying a system such as known from WO 2018/215272 A1 is beneficial in a situation in which disinfection of surfaces may contribute to avoiding contamination. Such a situation can occur in all kinds of environments, including public spaces, work environments and domestic settings. For example, if no disinfecting measures are applied, control buttons and touchscreens in public spaces constitute spots which involve a health risk, because transfer of infectious diseases may take place through the control buttons and touchscreens. Bacteria and viruses may be left on a control button or touchscreen by a first person, and subsequently get picked up by a next person, whereby a disease carried by the first person is spread. Public use of control buttons and touchscreens is common in view of the fact that control buttons and touchscreens are found in many types of devices such as elevators, ATM machines, order terminals, payment terminals and vending machines.

As explained in the foregoing, the system known from WO 2018/215272 A1 is effective in disinfection of surfaces, because radiation is coupled out of the waveguide element at the very positions on the waveguide element where there is any form of contamination such as virus or bacteria particles. In the context of the present invention, it is acknowledged that for various reasons, it may be so that the radiation exit window has one or more insufficiently irradiated areas, i.e. areas which are prevented from receiving the UV radiation at an intensity that is equal to or higher than a disinfecting minimum intensity. Hence, there is a need for developing measures aimed at removing or at least reducing the health/contamination risk that is related to the presence of the one or more insufficiently irradiated areas in the radiation exit window.

### SUMMARY OF THE INVENTION

It is an object of the invention to design a system comprising a radiation body in such a way that it is possible to have one or more insufficiently irradiated areas in the radiation exit window of the radiation body and to still avoid transfer of infectious diseases through the radiation exit window when the radiation exit window is touched by different human beings and/or different higher animals/pets. In the context of the invention, the radiation body does not necessarily need to be the same as the waveguide element disclosed in WO 2018/215272 A1, particularly does not necessarily need to be configured to have a radiation guiding function and to rely on the (total) internal reflection phenomenon in that respect, although the invention does cover the use of such a waveguide element.

In view of the foregoing, the invention provides a system comprising a radiation body comprising a radiation exit window, wherein the radiation body is configured to receive radiation that at least comprises UV radiation, wherein the radiation exit window is capable of allowing at least part of the radiation to pass to the exterior of the radiation body, wherein at least one area of the radiation exit window is prevented from receiving the UV radiation at an intensity that is equal to or higher than a disinfecting minimum intensity, and wherein the radiation exit window has an exterior surface structure that is configured to prevent contact between a body part of a human being or higher animal and the radiation exit window at the position of the at least one insufficiently irradiated area when the human being or higher animal touches the radiation exit window through the body part.

In the system according to the invention, the radiation exit window of the radiation body has at least one area at the position of which the extent to which the radiation exit window is disinfected is disinfected too small. Also, according to the invention, the radiation exit window is provided with an exterior surface structure that is configured to prevent contact between a body part of a human being or higher animal and the radiation exit window at the position of the at least one insufficiently irradiated area when the human being or higher animal touches the radiation exit window through the body part. The level of disinfection of the radiation exit window is minimal or even practically zero at the position of the at least one insufficiently irradiated area, but this still does not constitute a health risk to a human being or higher animal as the radiation exit window cannot be touched by a human being or higher animal through a body part at the position of the at least one insufficiently irradiated area anyway.

Examples of the above-mentioned body part of a human being or higher animal are a human finger and (another part of) a human hand. Examples of higher animals are mammals such as cats and dogs. In the present context, the term "body part" as applied to the human body is to be understood so as to only cover parts of the body which can be discerned at a first, general level of subdivision of the body, such as fingers, hands, arms and legs, and to not cover items included in the body as can be discerned at a micro and small scale level, such as hairs. Similarly, the term "body part" as applied to the animal body is to be understood so as to only cover parts of the body which can be discerned at a first, general level of subdivision of the body, such as paws, legs, nose and tail.

It is very practical if the exterior surface structure of the radiation exit window includes at least one indentation at least covering an insufficiently irradiated area. The area of the radiation exit window that is included in such an indentation is recessed relative to a main plane of the radiation exit window, which main plane may be generally straight or gradually curved, for example. When that area is sufficiently small relative to a touching body part, and has sufficient depth, the radiation exit window cannot be contacted by the body part at that area, as the general outline of the body part bridges the indentation and cannot be made to bulge outward into the indentation in such a way that contact would be obtained.

In many practical applications of the system according to the invention, it may be so that the radiation exit window is especially intended to be touched by human beings through their fingers. In view thereof, it may be advantageous if the above-mentioned at least one indentation is sufficiently small and sufficiently deep for preventing contact of a human finger to the radiation exit window at the position of the at least one indentation. For example, it may be practical if a diameter of the at least one indentation at the level of the main plane of the radiation exit window is in a range of 0.5 mm to 10 mm, or in a smaller range of 3 mm to 7 mm

The presence of the at least one insufficiently irradiated area in the radiation exit window may be the result of various causes, particularly various optical design principles as may be applied in the system. For example, it may be so that the system comprises at least one UV radiation source configured to emit the UV radiation that is used to disinfect the radiation exit window, and that the at least one UV radiation source is of a type that is configured to emit the UV radiation in a limited range of directions excluding directions towards an area of the radiation exit window that is thereby an insufficiently irradiated area of the radiation exit window. An example of such a UV radiation source is a UV radiation source that is configured to emit UV radiation only in directions which are substantially parallel to the main plane of the radiation exit window.

Another cause can be found in the possibility of designing the system so as to comprise at least one functional set of a UV radiation source configured to emit UV radiation and a UV shielding arrangement configured to at least partially block UV radiation in a path of the UV radiation emitted by the UV radiation source, at a position between the UV radiation source and an area of the radiation exit window that is thereby an insufficiently irradiated area of the radiation exit window. In such a functional set, the UV shielding arrangement serves to prevent an area of the radiation exit window that is directly in front of the UV radiation source from receiving the UV radiation at an intensity that is too high. The fact is that if the UV shielding arrangement would not be present, due to local outcoupling of the UV radiation source, the intensity at the area as mentioned may even be 1,000 higher than necessary for achieving the intended disinfecting effect. By applying the invention, it is achieved that it is possible to use the at least one functional set and to thereby have the advantage of preventing the creation of an area where the intensity of the UV radiation would be too high, while at the same time avoiding the introduction of a health risk at the position of the insufficiently irradiated area that is obtained in this way.

In a practical embodiment of the system according to the invention, the radiation body comprises a slab of material, and both the UV radiation source and the UV shielding arrangement of the at least one functional set are embedded in the slab of material. The material of the slab of material may be silicone, for example. It may further be practical if the UV shielding arrangement of the at least one functional set comprises a mirror configured to reflect UV radiation and/or if the at least one UV radiation source is configured to emit UV-C radiation. The at least one UV radiation source may comprise an LED, for example.

In case the system comprises a plurality of functional sets, it may be so that the functional sets are distributed throughout the system in any suitable way. In this respect, it is possible that the functional sets are positioned at a distance from each other in a regular pattern. In respect of embodiments of the system in which the UV radiation sources and the UV shielding arrangements of the respective functional sets are embedded in a slab of material of the radiation body, it is noted that the functional sets may be arranged in a pattern in which the functional sets are equally distributed throughout the slab of material, in a row or in a 2D array. In any case, the number of functional sets can be chosen freely and so as to be appropriate in a given situation.

A notable advantage of the invention is that it is possible to realize any appropriate sizing of the radiation body and radiation exit window and to still achieve disinfection of the radiation exit window as desired without being potentially harmful to human beings or higher animals. The invention allows for having a large-sized radiation body and radiation exit window in view of the fact that the UV radiation sources can be placed along the radiation body with, in principle, unlimited length. In this respect, it is noted that the invention provides a system in which the radiation exit window has a plurality of insufficiently irradiated areas, wherein the exterior surface structure of the radiation exit window includes a plurality of indentations at least covering the respective insufficiently irradiated areas. In case the system comprises a plurality of UV radiation sources, it may particularly be so that each of the insufficiently irradiated areas is associated with one of the UV radiation sources. The indentations may be positioned at a distance from each other in a regular pattern, and this may especially be appropriate when the system comprises a plurality of UV radiation sources and the UV radiation sources are positioned at a distance from each other in a regular pattern.

The invention also relates to an object comprising the system as described here before, wherein the radiation exit window of the radiation body of the system is arranged to be at an exterior side of the object. Examples of such an object include all of the examples mentioned in the foregoing in respect of the system known from WO 2018/215272 A1. In general, the objects may be selected from a group comprising domestic appliances, furniture, construction elements of buildings and vehicles, sanitary parts, medical devices, and components of all of the foregoing. The object may be a door knob, control button, touchscreen or the like that is especially intended to be touched regularly and temporarily by human beings, particularly different human beings.

The invention also relates to a method of adding a system as described here before to an existing object, wherein the radiation body of the system is applied to an exterior surface of the existing object. This may be done in any suitable way, including through gluing, by using fastening means or on the basis of a snap connection or form closure arrangement.

In respect of possible applications of the invention, the following further information is provided. It is common practice, in some cases even obliged practice, to indicate safety codes with devices. Among other things, a so-called IP Code has been developed, which is an international code that is used to classify and rate the degree of protection provided by mechanical casings and electrical enclosures against intrusion, dust, accidental contact, and water. In view of the fact that putting the invention to practice yields a system comprising a radiation exit window that can safely be touched by human beings or higher animals through a body part, it is achieved that the IP Code can be applied to the system according to the invention and/or any object equipped with at least one system according to the invention. In particular, when it comes to a first digit of the IP Code, level 2 is applicable, which represents a level of protection that is provided against access to hazardous parts by human fingers, generally by objects larger than 12.5 mm, or even level 3, which represents a level of protection that is provided against access to hazardous parts by objects larger than 2.5 mm.

The above-described and other aspects of the invention will be apparent from and elucidated with reference to the following detailed description of practical embodiments of a system comprising a radiation body, wherein a radiation exit window of the radiation body has at least one insufficiently irradiated area, and wherein the radiation exit window has an exterior surface structure that is configured to prevent contact between a body part of a human being or higher animal and the radiation exit window at the position of the at least one insufficiently irradiated area when the human being or higher animal touches the radiation exit window through the body part.

### BRIEF DESCRIPTION OF THE DRAWINGS

The invention will now be explained in greater detail with reference to the figures, in which equal or similar parts are indicated by the same reference signs, and in which:
Fig. 1 diagrammatically shows a system according to a first embodiment of the invention, comprising a radiation body and a functional set of a UV radiation source and a shielding arrangement;
Fig. 2 diagrammatically shows a portion of a system according to a second embodiment of the invention, comprising a radiation body and a number of functional sets; and
Fig. 3 diagrammatically shows a number of objects which can be equipped with a system according to the invention.

### DETAILED DESCRIPTION OF THE EMBODIMENTS

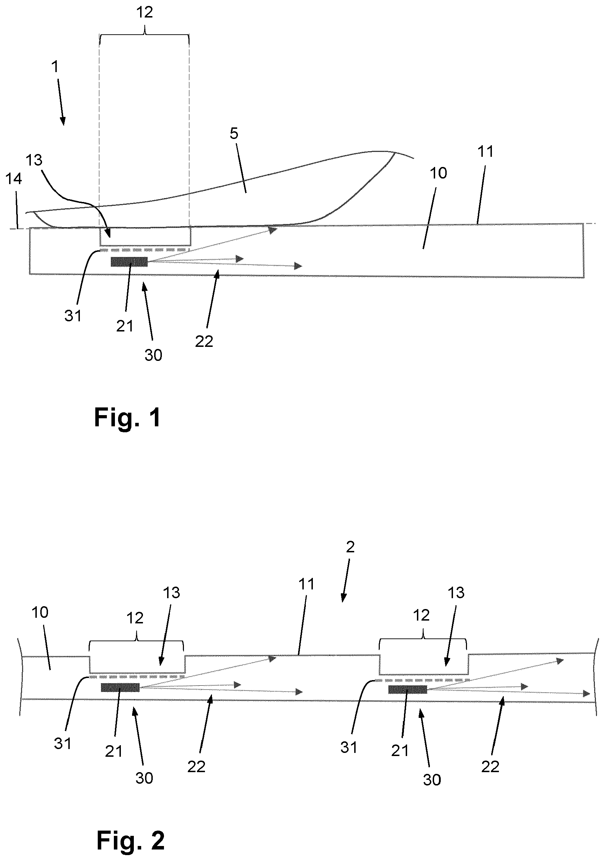

Fig. 1 diagrammatically shows a system 1 according to a first embodiment of the invention.

The system 1 comprises a radiation body 10 and a functional set 30 of a UV radiation source 21 and a shielding arrangement 31.

The radiation body 10 comprises a radiation exit window 11. Fig. 1 illustrates the practical options of the radiation body 10 comprising a slab of material and the radiation exit window 11 being of generally flat appearance.

The UV radiation source 21 of the functional set 30 is configured to emit UV radiation, in one or more directions, at least towards an interior side of the radiation exit window 11. In Fig. 1, the UV radiation is depicted by means of arrows 22. Fig. 1 illustrates the practical option of the UV radiation source 21 being embedded in the slab of material of the radiation body 10, wherein the slab of material is transparent to the UV radiation 22 provided by the UV radiation source 21. The UV radiation source 21 may be powered in any suitable way and may be electrically coupled to the mains or to a battery, for example.

The main purpose of having the UV radiation source 21 in the system 1 is keeping the exterior side of the radiation exit window 11 in a disinfected state. When bacteria or viruses end up on the exterior side of the radiation exit window 11, present in contaminated tiny droplets and/or grease/dirt, for example, the bacteria or viruses are killed or at least rendered inactive under the influence of UV radiation 22 exiting the radiation body 10 through the radiation exit window 11 at the position of the bacteria or viruses. In that way, spread of diseases via the radiation exit window 11 is prevented. This functionality of the system 1 is advantageous in many possible applications of the system 1, particularly applications in which the radiation exit window 11 is prone to be regularly and temporarily touched by human beings and/or higher animals/pets.

The shielding arrangement 31 of the functional set 30 is configured and arranged to at least partially block UV radiation 22 in a path of the UV radiation 22 emitted by the UV radiation source 21, at a position between the UV radiation source 21 and an area 12 of the radiation exit window 11, and is therefore also embedded in the slab of material of the radiation body 10. The fact is that the UV radiation source 21 is applied to provide the radiation body 10 with UV radiation 22 along the entire length and width of the slab of material of the radiation body 10. In view thereof, the intensity at which the UV radiation 22 is emitted by the UV radiation source 21 needs to a chosen such that a disinfecting effect on the exterior side of the radiation exit window 11 is obtained not only close to the UV radiation source 21, but also further away from the UV radiation source 21. As the material of the slab of material of the radiation body 10 absorbs the UV radiation 22, and possibly some of the UV radiation 22 is actually allowed to exit the radiation body 10 through the radiation exit window 11 for functional purposes, this means that a distribution of the intensity of the UV radiation 22 along the radiation exit window 11 is obtained, wherein the highest intensity is found in an area of the radiation exit window 11 that is closest to the UV radiation source 21, particularly directly in front of the UV radiation source 21. It may be so that the intensity at the area as mentioned can be expected to be so high that it is desirable to partially or completely block the UV radiation 22 in the path from the UV radiation source 21 to this area. Also, very close to the UV radiation source 21, the UV radiation 22 can escape directly from the radiation body 10. The shielding arrangement 31 is applied in such a way that the desired blocking action on the UV radiation 22 directly in front of the UV radiation source 21 is achieved, indeed.

The shielding arrangement 31 may be configured to block the UV radiation 22 by reflecting the UV radiation 22, for example, or may comprise some type of material that is highly absorbent of the UV radiation 22. In any case, as a consequence of applying the shielding arrangement 31, it may happen that an area 12 of the radiation exit window 11 is not disinfected to a sufficient extent. In view thereof, a measure is proposed that is aimed at achieving that it is not possible for a human being or a higher animal to contact the radiation exit window 11 at the position of this area 12, which is referred to as insufficiently irradiated area 12, by means of a body part. By putting this measure to practice, it does not matter that the radiation exit window 11 is not properly disinfected at the position of the insufficiently irradiated area 12, as the insufficiently irradiated area 12 cannot be touched anyway. This measure pertains to the exterior surface structure of the radiation exit window 11. In the shown example, this measure particularly involves having an indentation 13 of the radiation exit window 11 at the position of the insufficiently irradiated area 12. In Fig. 1, a human finger 5 is diagrammatically shown as being in a position of touching the radiation exit window 11 on an area including the insufficiently irradiated area 12. Due to the presence of the indentation 13, it is achieved that there cannot be any physical contact between the finger 5 and the radiation exit window 11 at the position of the indentation 13. Contact between the finger 5 and the radiation exit window 11 can only be established in an area of the radiation exit window 11 surrounding the insufficiently irradiated area 12, while the finger 5 bridges the indentation 13 without contacting the radiation exit window 11 at the position of the indentation 13. Thus, by choosing the design of the exterior surface structure of the radiation exit window 11 in a clever way, making use of a significant difference of dimensions of a touching object 5 and an insufficiently irradiated area 12 of the radiation exit window 11, a health risk for human beings and higher animals can be minimized or even practically reduced to zero, despite of the presence of the insufficiently irradiated area 12 as caused by the application of the UV shielding arrangement 31.

The indentation 13 may have any peripheral shape, such as a circular peripheral shape or a square peripheral shape. Also, the indentation 13 may have any suitable depth, wherein the indentation 13 may have a generally straight appearance in the depth direction, or a generally tapering appearance, for example. A practical example of a diameter of the indentation 13 at the level of a main plane 14 of the radiation exit window 11 is 5 mm, which does not alter the fact that any suitable sizing of the indentation 13 can be chosen in relation to the sizing of the UV radiation source 21 and the UV shielding arrangement 31, especially assuming that the latter sizing is generally in the millimeter range, and in relation to the sizing of the expected touching objects 5.

In general, the system according to the invention may comprise any appropriate number of UV radiation sources 21 and any appropriate number of associated shielding arrangements 31. In this respect, it is noted that Fig. 2 diagrammatically shows a portion of a system 2 according to a second embodiment of the invention, which system 2 includes a number of functional sets 30 of a UV radiation source 21 and a UV shielding arrangement 31. In view thereof, the system 2 will be referred to as extended system 2. The extended system 2 may in fact be regarded as comprising a number of the above-described systems 1 according to the first embodiment of the invention, in an integrated fashion, wherein the number may be chosen to have any appropriate value. For the sake of illustration, a portion of the extended system 2 including two functional sets 30 is shown in Fig. 2. On the basis of having several functional sets 30, it is possible to design the system 2 according to the invention with any possible dimensions, regardless of the UV absorbing characteristics of the material of the radiation body 10.

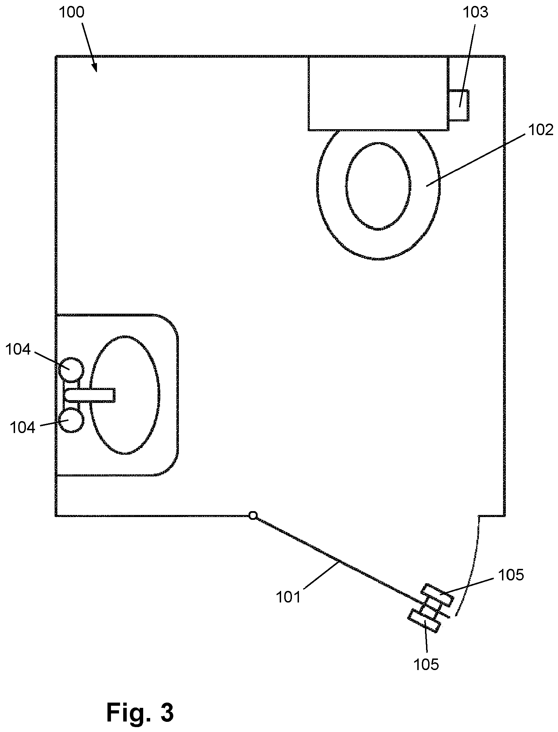

Fig. 3 diagrammatically shows a number of objects which can be equipped with the system 1, 2 according to the invention, particularly objects as can be found in a bathroom 100 and on a bathroom door 101, namely a toilet seat 102, a toilet flushing knob 103, tap knobs 104 and the door knobs 105 of the bathroom door. The shown objects are only a few of the many objects included in the scope of protection of the invention. The object 102, 103, 104, 105 can be of conventional design, in which case the system 1, 2 or at least the radiation body 10 of the system 1, 2 and components arranged in the radiation body 10 can be arranged on an exterior surface of the object 102, 103, 104, 105, or the object 102, 103, 104, 105 can be of adapted design, in which case the system 1, 2 or at least the radiation body 10 of the system 1, 2 and components arranged in the radiation body 10 can have a sunk arrangement in a member of the object 102, 103, 104, 105, for example, wherein the radiation exit window 11 of the radiation body 10 of the system 1, 2 can be flush with a surrounding part of an original exterior surface of the object 102, 103, 104, 105.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

Notable aspects of the invention are summarized as follows. In a system 1, 2 comprising a radiation body 10 configured to receive radiation that at least comprises UV radiation 22, at least one area 12 of a radiation exit window 11 of the radiation body 10 is prevented from receiving the UV radiation 22 at an intensity that is equal to or higher than a disinfecting minimum intensity. In order to avoid that a health risk arises from having the at least one area 12 that is not under the disinfecting influence of the UV radiation 22 to a sufficient extent, it is proposed to provide the radiation exit window 11 with an exterior surface structure that is configured to prevent contact between a body part 5 of a human being or higher animal and the radiation exit window 11 at the position of the at least one insufficiently irradiated area 12 when the human being or higher animal touches the radiation exit window 11 through the body part 5.

## Claims

1. A system (1, 2) comprising a radiation body (10) comprising a radiation exit window (11), wherein the radiation body (10) is configured to receive radiation that at least comprises UV radiation (22), wherein the radiation exit window (11) is capable of allowing at least part of the radiation to pass to the exterior of the radiation body (10), wherein at least one area (12) of the radiation exit window (11) is prevented from receiving the UV radiation (22) at an intensity that is equal to or higher than a disinfecting minimum intensity, and wherein the radiation exit window (11) has an exterior surface structure that is configured to prevent contact between a body part (5) of a human being or higher animal and the radiation exit window (11) at the position of the at least one insufficiently irradiated area (12) when the human being or higher animal touches the radiation exit window (11) through the body part (5).

2. The system (1, 2) according to claim 1, wherein the exterior surface structure of the radiation exit window (11) includes at least one indentation (13) at least covering an insufficiently irradiated area (12).

3. The system (1, 2) according to claim 2, wherein the at least one indentation (13) is sufficiently small and sufficiently deep for preventing contact of a human finger (5) to the radiation exit window (11) at the position of the at least one indentation (13).

4. The system (1, 2) according to claim 2 or 3, wherein a diameter of the at least one indentation (13) at the level of a main plane (14) of the radiation exit window (11) is in a range of 0.5 mm to 10 mm

5. The system (1, 2) according to any of claims 1-4, comprising at least one UV radiation source (21) configured to emit UV radiation (22) in a limited range of directions excluding directions towards an area (12) of the radiation exit window (11) that is thereby an insufficiently irradiated area (12) of the radiation exit window (11).

6. The system (1, 2) according to claim 5, wherein the at least one UV radiation source (21) is configured to emit UV radiation (22) only in directions which are substantially parallel to a main plane (14) of the radiation exit window (11).

7. The system (1, 2) according to any of claims 1-6, comprising at least one functional set (30) of a UV radiation source (21) configured to emit UV radiation (22) and a UV shielding arrangement (31) configured to at least partially block UV radiation (22) in a path of the UV radiation (22) emitted by the UV radiation source (21), at a position between the UV radiation source (21) and an area (12) of the radiation exit window (11) that is thereby an insufficiently irradiated area (12) of the radiation exit window (11).

8. The system (1, 2) according to claim 7, wherein the radiation body (10) comprises a slab of material, and wherein both the UV radiation source (21) and the UV shielding arrangement (31) of the at least one functional set (30) are embedded in the slab of material.

9. The system (1, 2) according to claim 7 or 8, wherein the UV shielding arrangement (31) of the at least one functional set (30) comprises a mirror configured to reflect UV radiation (22).

10. The system (1, 2) according to any of claims 5-9, wherein the at least one UV radiation source (21) is configured to emit UV-C radiation.

11. The system (1, 2) according to any of claims 5-10, wherein the at least one UV radiation source (21) comprises an LED.

12. The system (2) according to any of claims 1-11, wherein the radiation exit window (11) has a plurality of insufficiently irradiated areas (12), wherein the exterior surface structure of the radiation exit window (11) includes a plurality of indentations (13) at least covering the respective insufficiently irradiated areas (12), and wherein the indentations (13) are positioned at a distance from each other in a regular pattern.

13. An object (102, 103, 104, 105) comprising the system (1, 2) according to any of claims 1-12, wherein the radiation exit window (11) of the radiation body (10) of the system (1, 2) is arranged to be at an exterior side of the object (102, 103, 104, 105).

14. The object (102, 103, 104, 105) according to claim 13, selected from a group comprising domestic appliances, furniture, construction elements of buildings and vehicles, sanitary parts, medical devices, and components of all of the foregoing.

15. Method of adding a system (1, 2) according to any of claims 1-12 to an existing object (102, 103, 104, 105), wherein the radiation body (10) of the system (1, 2) is applied to an exterior surface of the existing object (102, 103, 104, 105).
